# EUROPEAN PATENT APPLICATION

(11) **EP 1 944 017 A2**
(43) Date of publication of application: **16.07.2008**
(21) Application number: 08380003.7
(22) Date of filing: 09.01.2008
(51) Int. Cl.: A61K 9/00, A61K 9/20, A61K 31/366, A61K 31/40, A61K 31/401, A61K 31/44

(54) **Rapidly disintegrating tablet in the oral cavity**

(30) Priority: 11.01.2007 ES 200700110
(71) Applicant: Tedec-Meiji Farma, S.A., 28802 Alcalá de Henares Madrid (ES)
(72) Inventor: Nishizawa, Shinichi, 28002 Alcalá de Henares (Madrid) (ES); De Castro Torres, Juan Luis, 28002 Alcalá de Henares (Madrid) (ES)
(74) Representative: Rodriguez Perez, Jesus

(57) **Abstract**

The invention discloses an intraorally rapidly disintegrable tablet containing a drug, crystalline cellulose and a sugar alcohol in a specific proportion, without the presence of any disintegrating agent. The intraorally rapidly disintegrable tablet according to the present invention disintegrates rapidly when placed in the mouth with a small amount of water and is smoothly swallowed without leaving a gritty sensation in the oral cavity, also presenting excellent storage stability.

## Description

### Technical field of the invention

The present invention relates to a tablet that disintegrates rapidly in the oral cavity, which means that it disintegrates very rapidly in the mouth when taken with a minimal amount of water.

### Prior art

Taking tablets can be difficult in the case of elderly people or children due to their more limited swallowing capacity. There is therefore a need to develop pharmaceutical preparations that are easy to take when administered to elderly people, children or adults with difficulty swallowing, pharmaceutical preparations that also disintegrate rapidly in the mouth and are easy to swallow without leaving a gritty sensation in the oral cavity. Such pharmaceutical preparations are already known.

For example, Japanese Sho Patent Publication No. 62-50445 discloses a pharmaceutical preparation that disintegrates rapidly in the oral cavity that can be produced by inserting into the cavity of the PTP (press-through package) a previously prepared solution containing a drug, a polymer such as gelatine and mannitol, and cryogenically drying the solution. WO 93/12769 discloses a pharmaceutical preparation that rapidly disintegrates in the oral cavity that can be produced by filling a mould with a suspension containing a drug, a saccharide such as lactose or mannitol and an aqueous agar solution, solidifying the suspension to form a gelatine and drying the gelatine thus produced. Japanese Laid-Open Patent No. 5-271054 discloses a method of producing a tablet that dissolves in the oral cavity that has a porous structure, its porosity being approximately 20 to 80%, the method comprising compression-moulding a mixture containing a drug, a saccharide and a barely sufficient amount of water to moisten the surface of the particles, at a pressure of 3 to 160 kg/cm². Japanese Patent Publication No. 8-30005 presents a disintegrating tablet containing a sparingly or slowly soluble pharmaceutically active substance and a disintegrating agent, in addition to special filler particles (filler particles coated with a layer of a colloid or pseudo-colloid, or fused granules of a slowly-dissolving material that are produced by melting, cooling and agglomeration). WO 97/38679 discloses a pharmaceutical preparation that rapidly disintegrates in the oral cavity that can be produced by filling a mould with a mixture solution containing a drug, a filler such as mannitol, a binder and other auxiliary substances, and cryogenically drying the mixture solution thus produced. Japanese Laid-Open Patent No. 9-48726 discloses a tablet that rapidly disintegrates in the oral cavity that can be produced by moistening a mixture containing a drug and substances such as saccharides, a sugar alcohol and a polymer that is highly soluble in water, by moistening the mixture at a low density and drying it. However, it can be considered that these conventional pharmaceuticals preparations present various disadvantages in terms of their production and storage, e.g. they are not very hard, and they involve a complicated production process.

Japanese Laid-Open Patent No. 8-27033, for its part, discloses a solid pharmaceutical preparation containing a medicinal component and erythritol in a homogeneous state. Japanese Laid-Open Patent No. 8-27033 cites that the solid pharmaceutical preparation has properties such as the refreshing flavour of the erythritol, a good palatability, low moisture absorption capacity and a good stability, and furthermore it hardly absorbs moisture and is very stable and easy to take. Moreover, Japanese Laid-Open Patent No. 8-27033 discloses that the solid pharmaceutical preparation presents excellent mechanical properties with regard to its capacity to be made into tablets or compacted, and therefore its easy production in tablet-form. However, Japanese Laid-Open Patent No. 8-27033 does not include any description of the hardness and the in-mouth disintegration capacity of the solid pharmaceutical preparation.

### Problem to be solved by the invention

Bearing in mind the aforementioned circumstances, it is clear that there is a need for an intraorally rapidly disintegrable tablet that is easy to produce and that disintegrates rapidly in the mouth, but that also has the necessary hardness for its production and storage. The intraorally rapidly disintegrable tablets must also ensure that the sensation that the drug leaves in the mouth is more pleasant than in the case of tablets disclosed in the prior art.

### Summary of the invention

The present invention relates to the following points 1 to 7:
1. An intraorally rapidly disintegrable tablet comprising a drug, crystalline cellulose, a sugar alcohol and not containing a disintegrating agent, which is produced by compression-moulding a mixture consisting of the drug, the crystalline cellulose and the sugar alcohol, characterised in that the crystalline cellulose and the sugar alcohol are mixed in a proportion of 5:5 to 3.5:6.5 by weight.
2. Intraorally rapidly disintegrable tablet according to 1, which is produced by mixing the drug, the crystalline cellulose and the sugar alcohol to produce a mixture and directly compression-moulding said mixture.
3. Intraorally rapidly disintegrable tablet according to 1, which is produced by mixing the drug, the crystalline cellulose and the sugar alcohol to produce a mixture, subjecting the mixture to wet granulation to produce granules and compression-moulding said granules.
4. Intraorally rapidly disintegrable tablet according to 3, which is produced by mixing the drug, part of the crystalline cellulose and the sugar alcohol to produce a mixture, subjecting the mixture to wet granulation to produce granules, mixing the rest of the crystalline cellulose with said granules and compression-moulding these mixtures with the rest of the crystalline cellulose.
5. Intraorally rapidly disintegrable tablet according to 1, which is produced by mixing the drug and the sugar alcohol to produce a mixture, subjecting the mixture to wet granulation to produce granules, mixing the crystalline cellulose with said granules and compression-moulding them together with the crystalline cellulose.
6. Intraorally rapidly disintegrable tablet according to any of 1 to 5, wherein the sugar alcohol is selected from at least one of the group consisting of mannitol, erythritol and xylitol.
7. Intraorally rapidly disintegrable tablet according to any of 1 to 5, wherein the sugar alcohol is mannitol.

### Detailed description of the invention

An intraorally rapidly disintegrable tablet according to the present invention contains crystalline cellulose and a sugar alcohol in a specific proportion, it does not contain any disintegrating agent and it is produced by compression moulding. The characteristics of the present invention will be disclosed below. Firstly, examples of sugar alcohols to be used in the present invention include mannitol, erythritol and xylitol. These sugar alcohols may be used alone or as a combination of two or more thereof. Preferably, mannitol is used.

The crystalline cellulose and the sugar alcohols to be used in the present invention are not particularly limited, as they are the ones that have a suitable quality for use in pharmaceutical preparations in general. As these materials can take different forms, the proportion of the mixture of crystalline cellulose and the sugar alcohol or the combination of sugar alcohols may be appropriately varied to produce an excellent pharmaceutical preparation. Moreover, the amount of these materials to be mixed can easily be determined. For example, by appropriately mixing the desired components with a drug to produce a mixture, subjecting the mixture to compression moulding and checking the hardness and the disintegration capacity of the resulting preformed material it is possible to easily determine whether or not the amount of the desired components in the mixture is suitable.

The intraorally rapidly disintegrable tablet according to the present invention is characterised in that the crystalline cellulose and the sugar alcohol are mixed in a proportion of 5:5 to 3.5:6.5, preferably 5:5 to 4:6. If the proportion of crystalline cellulose:sugar alcohol exceeds the upper limit, after the tablet has disintegrated in the oral cavity it may leave a gritty feeling and a sensation of foreign matter that is inherent to crystalline cellulose, which is not advisable for easy ingestion. Furthermore, if the proportion of crystalline cellulose:sugar alcohol is less than the lower limit, the forming and disintegration characteristics are reduced, thus causing problems of form and an increase in the time required for its disintegration in the mouth.

In general, for the tablets to present the desired disintegration and solubility properties it is necessary for them to include a disintegrating agent (e.g. croscarmellose sodium, sodium carboxymethyl starch, low-substituted hydroxypropyl cellulose). However, contrary to expectations, the present inventors have found that it is possible to produce a tablet with suitable disintegration characteristics without using a disintegrating agent and by compression-moulding a mixture containing crystalline cellulose and a sugar alcohol in a specific proportion. When a general-use disintegrating agent is added to the intraorally rapidly disintegrable tablet of the present invention, the time required for disintegration increases rather than decreases (see Example 6). Moreover, the addition of a disintegrating agent does not provide any advantage for the intraorally rapidly disintegrable tablet of the present invention.

The drug to be used in the intraorally rapidly disintegrable tablet of the present invention is not particularly limited, provided that it can be administered orally and does not have an unpleasant flavour, e.g. bitter or sharp, to avoid said flavour in the mouth making it difficult to take. Examples of such drugs include antibiotics, chemotherapy drugs, hypnotic and anxiolytic sedatives, anti-epileptic drugs, analgesic, antipyretic and anti-inflammatory agents, drugs for the treatment of Parkinson's, psychotropic drugs, musculoskeletal relaxants, drugs that act on the autonomic nervous system, spasmolytic drugs, cardiotonic drugs, anti-arrhythmic drugs, diuretics, hypotensive agents, capillary protectors, vasoconstrictors, vasodilators, anti-hyperlipidaemic drugs, antitussive expectorants, bronchodilators, anti-diarrhoea drugs, drugs for intestinal function, for peptic ulcers, for the stomach, antacids, cholagogues, gastrointestinal drugs, vitamins, nutritional supplements, drugs for liver disease, anti-gout drugs, anti-diabetic drugs, anti-tumour drugs, anti-histamines, pharmaceutical formulations and drugs for osteoporosis.

The proportion of crystalline cellulose and a sugar alcohol in the powdered mixture is not particularly limited. However, bearing in mind the form and disintegration characteristics, the amount of drug to be mixed is 0.0001 to 2 parts by weight, preferably 0.005 to 0.1 parts by weight, in particular 0.001 to 0.01 parts by weight for each part by weight of the powdered mixture of crystalline cellulose and the sugar alcohol.

If necessary, the intraorally rapidly disintegrable tablet of the present invention may also contain different additives that are of general use in the production of tablets. These additives may be used separately or in a combination of two or more thereof in any proportion. Examples of such additives are binders, fluidising agents, lubricants, flavourings, sweeteners, correctors and colourings. Examples of binders include powdered gum arabic, gelatine, pullulan and polyvinyl alcohol. Of these, it is preferable to use binders in powder form that have weak bonds, e.g. pullulan and polyvinyl alcohol. Examples of fluidising agents are silicon dioxide hydrate and silicic anhydride. Examples of lubricants include magnesium stearate, calcium stearate, talc and sucrose fatty acid esters. Examples of flavourings are orange, strawberry, yoghurt, vanilla and menthol. Examples of sweeteners include aspartame, saccharine, dipotassium glycyrrhizinate and stevia. Examples of correctors are citric acid, tartaric acid, maleic acid, ascorbic acid, sodium citrate, sodium chloride and 1-menthol. Examples of colourings include food dyes such as Red Food Dye No. 3, Yellow Food Dye No. 5 and Blue Food Dye No. 1. In general, these components may be used separately or in a combination of two or more thereof and in any amount, provided that the disintegration and form characteristics of the rapidly disintegrating tablet in the oral cavity of the present invention are not affected. The combination of crystalline cellulose/mannitol/silicic anhydride/aspartame/strawberry flavouring could be given as an example.

According to a method of preparing the intraorally rapidly disintegrable tablet of the present invention, a drug, crystalline cellulose and a sugar alcohol are mixed and the amount is adjusted in the usual way, producing a powder for compression moulding. In the present invention, the particle sizes of the drug and the additive are not particularly limited, but, in terms of a better sensation when taking the tablet, they should preferably be small. The method can include a wet granulation step, if necessary. In this case, fluid bed granulation, high-speed stirring granulation or a similar type of granulation is carried out after the drug, the crystalline cellulose and the sugar alcohol have been mixed in the normal way. A suitable lubricant is then added to the granules that have been produced to prepare a powder for compression moulding. It is thus possible to significantly improve the flowability without having to add a fluidising agent, also eliminating problems when it comes to forming the tablets (see Example 3). If wet granulation is performed, it is not always necessary to add the whole amount of crystalline cellulose before granulation, and part or all of the crystalline cellulose can finally be added to the granules produced by granulation.

The powder for compression moulding thus produced is fed into a mould and then compression-moulded at a pressure of 200 to 1,500 kg, preferably 400 to 800 kg of pressure. If the pressure is less than the value of the lower limit, the resulting tablet has an inadequate hardness that is not sufficient to allow it to be handled. However, if the pressure exceeds the upper limit established above, the disintegration time of the resulting tablet increases, which is a disadvantage. The intraorally rapidly disintegrable tablet according to the invention can be prepared in any tablet shape: round, oval, oblong.

The intraorally rapidly disintegrable tablet according to the present invention disintegrates rapidly when placed in the mouth with a small amount of water (5 to 20 ml) and is smoothly swallowed without leaving a gritty sensation in the oral cavity. The intraorally rapidly disintegrable tablet according to the present invention normally dissolves in the mouth in approximately 1 to 30 seconds, preferably in approximately 2 to 20 seconds, in particular in approximately 8 to 16 seconds. Although it is known that, in general, a tablet should have a hardness of approximately 3.5 to 7 kg (value measured in a tablet hardness test) and to prevent problems, the hardness of the intraorally rapidly disintegrable tablet according to the present invention is approximately 2 to 20 kg, preferably approximately 3 to 15 kg. It is also known that, in general, the correct friability for a tablet is 0.8% or less; however, the friability of the intraorally rapidly disintegrable tablet according to the present invention is well below said value (see Example 2).

If the powdered mixture consisting of a sugar alcohol and the crystalline cellulose is subjected to wet granulation (e.g. fluid bed or high-speed stirring granulation) in the production process of the intraorally rapidly disintegrable tablet according to the present invention, the time required for disintegration of the resulting tablet increases over time after the tablet-forming process is completed when erythritol or xylitol are used therein. However, such phenomena do not occur when mannitol is used (see Example 4). Therefore, the use of mannitol is preferable, as it favours stability. However, it has been found that if a drug, part of the crystalline cellulose and a sugar alcohol are mixed and the mixture is subjected to wet granulation to produce granules, and then the rest of the crystalline cellulose is finally added to the granules that have been produced, or if a drug and a sugar alcohol are mixed and the mixture is subjected to wet granulation to produce granules and the total amount of crystalline cellulose is finally added to the granules that have been produced, the time required for disintegration of the resulting tablet does not increase over time, even when erythritol or xylitol are used (see Example 5). In these cases, the amount of crystalline cellulose to be finally added is preferably 75% by weight or more than the total amount of crystalline cellulose.

As has been described above, the intraorally rapidly disintegrable tablet according to the present invention is easy to produce and is sufficiently strong for production and storage and has excellent stability in long-term storage conditions. Furthermore, the intraorally rapidly disintegrable tablet according to the present invention disintegrates rapidly in the mouth and can be used as a pharmaceutical preparation that is easy for elderly people and children to take, also being a safe preparation for adults. As in the case of a conventional oral pharmaceutical preparation containing the same drug, the intraorally rapidly disintegrable tablet according to the present invention may be used to prevent and treat a variety of diseases.

According to the present invention, it is possible to provide an intraorally rapidly disintegrable tablet that is excellent in terms of form and hardness and at the same time has a good disintegration capacity. In particular, the intraorally rapidly disintegrable tablet according to the present invention constitutes an excellent oral pharmaceutical preparation that disintegrates rapidly when placed in the mouth with a small amount of water and can be swallowed without leaving a gritty sensation. Moreover, the intraorally rapidly disintegrable tablet is physically rigid and also easy to handle. For these reasons, the intraorally rapidly disintegrable tablet can be used to treat or prevent a variety of diseases of patients, in particular geriatric or paediatric patients, by changing only the drug contained in the tablet. Furthermore, the intraorally rapidly disintegrable tablet according to the present invention is economical and efficient to produce, as it does not require a disintegrating agent or any specialised equipment. The intraorally rapidly disintegrable tablet according to the present invention contains a sugar alcohol that has a strong and refreshing sweet flavour, which also makes it easy to take.

### Examples

The present invention is described below in detail with reference to the following examples, without the invention being limiting thereto.

### Example 1

40 g of crystalline cellulose and 60 g of a sugar alcohol (any selected from D-mannitol, xylitol and erythritol) were mixed to produce a mixture, and said mixture was subjected to a normal procedure of adjustment to produce a fine powder. 500 mg of the fine powder was then placed in a mould with an 11.3 mm diameter and it was compressed at a pressure of approximately 1,200 kg. Meanwhile, a pharmaceutical preparation was prepared as a control in the manner described above, using sucrose instead of the sugar alcohol. A comparison was carried out of the hardness, disintegration and in-mouth disintegration time of the two pharmaceuticals preparations. It must be mentioned that the hardness was measured using a tablet hardness test (Schleuniger), the disintegration was measured in water according to the disintegration test described in Japanese Pharmacopoeia XIII and the in-mouth disintegration time was determined by measuring the time that it took for a tablet to completely disintegrate in 20 ml of water in the mouth.

The results are shown in Table 1. The results show that the intraorally rapidly disintegrable tablet according to the present invention has an excellent hardness and disintegration capacity.

**Table 1**

| Excipient | Hardness (kg) | Disintegration (min) | In-mouth disintegration time |
|---|---|---|---|
| Mannitol | 11.3 | 0.2 | 12 seconds |
| Erythritol | 6.1 | 0.2 | 9 seconds |
| Xylitol | 8.8 | 0.2 | 11 seconds |
| Sucrose | 11.7 | 1.0 | 1 minute or more |

### Example 2

120.0 g of crystalline cellulose and 179.1 g of D-mannitol were mixed to produce a mixture, which was subjected to granulation with water using a fluid bed granulator and the granules thus produced were dried sufficiently. 0.9 g of magnesium stearate was finally mixed with the granules, preparing the granules to form tablets. 250 mg of the granules for tablets were then placed in a mould with a 9 mm diameter and they were subjected to compression moulding to produce the tablet. Several different tablets were thus prepared by compression moulding at different pressures (i.e. at different tablet production pressures). Comparisons were made of the hardness, disintegration and in-mouth disintegration time and the friability of these different tablets. The hardness, disintegration and in-mouth disintegration time were measured in the same way as in Example 1. The friability was determined using a tablet friability tester with 20 of them in the normal manner. Specifically, the 20 tablets were made to rotate at 100 revolutions in the friability tester and the weight loss due to abrasion was measured.

The results are shown in Table 2. The friability results show that the intraorally rapidly disintegrable tablet according to the present invention has an excellent form and is physically strong and also easy to handle.

**Table 2**

| Tablet formation pressure (t) | Hardness (kg) | Disintegration (min) | In-mouth disintegration time | Friability (%) |
|---|---|---|---|---|
| 0.5 | 3.8 | 0.2 | 8 seconds | 0.369 |
| 0.75 | 6.0 | 0.3 | 10 seconds | 0.030 |
| 1.0 | 7.6 | 0.3 | 12 seconds | 0.012 |
| 1.5 | 9.8 | 0.3 | 13 seconds | 0.008 |

### Example 3

80 g of crystalline cellulose and 120 g of erythritol were mixed to produce a mixture, which was subjected to granulation using water in a fluid bed granulator. The granules thus produced were dried sufficiently to prepare a suitable powder for tablets. Meanwhile, 80 g of crystalline cellulose and 120 g of erythritol were mixed to produce a mixture, the amount of which was adjusted in the normal way to prepare a powder for control tablets. The flowability of the two powders for tablets was compared. It should be noted that the flowability was measured according to a method using an instrument to measure the angle of repose.

The results are shown in Table 3. It can be inferred from these results that the flowability significantly improves when granulation is carried out.

**Table 3**

| | Angle of repose |
|---|---|
| Granules | 38°40' |
| Mixture in powder form | 55°35' |

### Example 4

98.8 g of crystalline cellulose and 148.2 g of a sugar alcohol (any selected from D-mannitol, xylitol and erythritol), 1 g of aspartame and 1 g of ascorbic acid were placed in a fluid bed granulator, and the mixture was subjected to granulation with water. The granules thus produced were dried sufficiently. Then 0.75 mg of magnesium stearate and 0.25 mg of lemon flavouring were mixed with 249 mg of the granules and the mixture that was produced was placed in a mould with a 9 mm diameter. The mixture was compressed at a pressure of approximately 400 kg to produce a tablet. The hardness and in-mouth disintegration time of the tablet was compared just after its compression and after having been stored at 25°C, 61% relative humidity, for a week. The hardness and in-mouth disintegration time were measured as in Example 1.

The results are shown in Table 4. In the case of the pharmaceutical preparation with D-mannitol, the hardness did not increase and the disintegration time did not change, even after being stored for 1 week. However, in the case of the pharmaceutical preparation with erythritol or xylitol, the disintegration time increase after being stored for 1 week. The results show that when a pharmaceutical preparation is produced by wet granulation (fluid bed granulation or high-speed stirring granulation) using a powdered mixture of a sugar alcohol and crystalline cellulose, if the pharmaceutical preparation contains mannitol it has greater stability in terms of disintegration.

**Table 4**

| Excipient | Hardness (kg) | | In-mouth disintegration time | |
|---|---|---|---|---|
| | Just after forming the tablet | After 1 week | Just after forming the tablet | After 1 week |
| Mannitol | 3.7 | 3.6 | 11 seconds | 11 seconds |
| Erythritol | 3.1 | 4.8 | 10 seconds | 21 seconds |
| Xylitol | 3.5 | 5.1 | 13 seconds | 32 seconds |

### Example 5

148.2 g of a sugar alcohol (erythritol or xylitol), 1 g of aspartame and 1 g of ascorbic acid were placed in a fluid bed granulator and the mixture was subjected to granulation with water. The granules thus produced were dried sufficiently. Then 150.2 mg of the granules were mixed with 98.8 mg of crystalline cellulose, 0.75 mg of magnesium stearate and 0.25 mg of lemon flavouring and the mixture thus produced was placed in a mould with a 9 mm diameter. The mixture was compressed at a pressure of approximately 400 kg to produce a tablet. The hardness and in-mouth disintegration time of the tablet was compared just after its compression and after having been stored at 25°C, 61% relative humidity, for a week. The hardness and in-mouth disintegration time were measured as in Example 1.

The results are shown in Table 5. The results show that if the crystalline cellulose is finally added to the granules produced by granulation, the hardness and disintegration time do not increase, even after being stored for 1 week.

**Table 5**

| Excipient | Hardness (kg) | | In-mouth disintegration time | |
|---|---|---|---|---|
| | Just after forming the tablet | After 1 week | Just after forming the tablet | After 1 week |
| Erythritol | 3.4 | 3.4 | 9 seconds | 9 seconds |
| Xylitol | 3.6 | 3.7 | 11 seconds | 12 seconds |

### Example 6

200 g of a powdered mixture produced by mixing crystalline cellulose and D-mannitol in a proportion of 4:6 was subjected to granulation in water in a fluid bed granulator. The granules thus produced are dried sufficiently. Then 0.3% of magnesium stearate was finally mixed with the granules to prepare granules for tablets. 250 mg of the granules for tablets were placed in a mould with a 9 mm diameter and compressed at a pressure of approximately 800 kg to produce a tablet. Meanwhile, 5.0% of a disintegrating agent (one selected from croscarmellose sodium, sodium carboxymethyl starch and low-substituted hydroxypropyl cellulose) and 0.3% of magnesium stearate was finally mixed with the aforementioned granules for tablets, containing crystalline cellulose and D-mannitol, and 250 mg of the granules for tablets was subjected to compression moulding in the manner described above to produce a control tablet. The hardness and in-mouth disintegration time were measured as in Example 1 and the different tablets were compared.

The results are shown in Table 6. The results show that the addition of a disintegrating agent increases the disintegration time more than reducing it and that it is not advantageous to add the disintegrating agent.

**Table 6**

| Disintegrating agent | Hardness (kg) | In-mouth disintegration time |
|---|---|---|
| None (Tablet according to the present invention) | 5.0 | 10 seconds |
| Croscarmellose sodium | 4.9 | 24 seconds |
| Sodium carboxymethyl starch | 4.2 | 22 seconds |
| Low-substituted hydroxypropyl cellulose | 4.5 | 28 seconds |

In the above Examples 1 to 6, as well as the advantages deriving from the data and considerations seen in said examples, the high degree of palatability of the tablet of the present invention greatly improves the sensation of freshness, therefore making it easier to take. In particular, the sensation of the tablet in the mouth according to the invention is much more pleasant than in the case of other similar tablets in the prior art, e.g. those comprising lactose rather than a sugar alcohol, as is the case in the present invention.

### Production example 1

2 mg of ethyl idelazepate as the active substance, 140 mg of crystalline cellulose, 205.2 mg of xylitol, 0.7 mg of silicic anhydride, 1.75 mg of aspartame and 0.35 mg of strawberry flavouring were mixed together and ground in a mortar. 350 mg of the fine powder thus produced was then placed in a mould with a 10 mm diameter and compressed at a pressure of approximately 750 kg to produce an intraorally rapidly disintegrable tablet according to the present invention.

### Production example 2

0.4 mg of rolipram as the active substance, 140 mg of crystalline cellulose, 206.8 mg of D-mannitol, 0.7 mg of anhydrous silicon dioxide, 1.75 mg of aspartame and 0.35 mg of orange flavouring were mixed together and ground in a mortar. 350 mg of the fine powder thus produced was then placed in a mould with a 10 mm diameter and compressed at a pressure of approximately 500 kg to produce an intraorally rapidly disintegrable tablet according to the present invention.

### Production example 3

2.5 mg of sodium picosulphate as the active substance, 166.8 mg of crystalline cellulose, 60 mg of D-mannitol, 60 mg of erythritol, 60 mg of xylitol and 0.7 mg of silicic anhydride were mixed together and ground in a mortar. 350 mg of the fine powder thus produced was then placed in a mould with a 10 mm diameter and compressed at a pressure of approximately 400 kg to produce an intraorally rapidly disintegrable tablet according to the present invention.

### Production example 4

5 g of enalapril maleate as the active substance, 23.4 g of crystalline cellulose, 140.4 g of erythritol, 5 g of aspartame and 5 g of ascorbic acid were mixed together to produce a mixture, which was subjected to granulation with water in a fluid bed granulator. The granules thus produced were dried sufficiently. 70.2 g of crystalline cellulose, 0.75 g of magnesium stearate and 0.25 g of vanilla flavouring were then mixed with 178.8 g of the granules to produce a mixture. The mixture was formed into tablets using a rotary tabletting machine with a bevelled edge punch and a 9 mm diameter at a pressure of 400 kg to provide intraorally rapidly disintegrable tablets according to the present invention, each weighing 250 mg.

### Production example 5

5 g of enalapril maleate as the active substance, 93.6 g of crystalline cellulose, 140.4 g of D-mannitol, 5 g of aspartame and 5 g of citric acid were mixed together to produce a mixture, which was subjected to granulation with water in a granulator. The granules thus produced were dried sufficiently. 0.75 g of magnesium stearate and 0.25 g of vanilla flavouring were then mixed with 249.0 g of the granules to produce a mixture. The mixture was formed into tablets using a rotary tabletting machine with a bevelled edge punch and a 9 mm diameter at a pressure of 400 kg to provide intraorally rapidly disintegrable tablets according to the present invention, each weighing 250 mg.

### Production example 6

5 g of simvastatin as the active substance, 93.6 g of crystalline cellulose, 140.4 g of D-mannitol, 5 g of aspartame and 5 g of citric acid were fed into a high-speed stirring granulator (high-speed mixer) and were mixed and subjected to granulation using a 90% ethanol aqueous solution. The granules thus produced were dried sufficiently using a hot air drier. 0.75 g of magnesium stearate and 0.25 g of orange flavouring were then mixed with 249.0 g of the granules to produce a mixture. The mixture was formed into tablets using a rotary tabletting machine with a bevelled edge punch and a 9 mm diameter at a pressure of 400 kg to provide intraorally rapidly disintegrable tablets according to the present invention, each weighing 250 mg.

### Production example 7

2 g of d-chlorpheniramine maleate as the active substance, 10 g of phenylephrine hydrochloride, 0.2 g of belladonna alkaloids and 141.78 g of erythritol were mixed together to produce a mixture, which was subjected to granulation with water in a fluid bed granulator. The granules thus produced were dried sufficiently. 94.52 g of crystalline cellulose, 0.75 g of magnesium stearate and 0.75 g of 1-menthol were then mixed with 153.98 g of the granules to produce a mixture. The mixture was formed into tablets using a rotary tabletting machine with a bevelled edge punch and a 9 mm diameter at a pressure of 500 kg to provide intraorally rapidly disintegrable tablets according to the present invention, each weighing 250 mg.

## Claims

1. Intraorally rapidly disintegrable tablet comprising a drug, crystalline cellulose, a sugar alcohol and no disintegrating agent, which is produced by compression moulding of a mixture consisting of a drug (API), crystalline cellulose and a sugar alcohol, **characterised in that** the crystalline cellulose and the sugar alcohol are mixed in a weight proportion of 5:5 to 3.5:6.5.

2. Intraorally rapidly disintegrable tablet according to claim 1, **characterised in that** it is produced by mixing the drug, the crystalline cellulose and the sugar alcohol to produce a mixture and then directly compression-moulding said mixture.

3. Intraorally rapidly disintegrable tablet according to claim 1, **characterised in that** it is produced by mixing the drug, the crystalline cellulose and the sugar alcohol to produce a mixture, subjecting the mixture to wet granulation to produce granules and then compression-moulding the granules.

4. Intraorally rapidly disintegrating tablet according to claim 3, **characterised in that** it is produced by mixing the drug, part of the crystalline cellulose and the sugar alcohol to produce a mixture, subjecting the mixture to wet granulation to produce granules, mixing the rest of the crystalline cellulose with the granules and then compression-moulding the granules that have been mixed with the rest of the crystalline cellulose.

5. Intraorally rapidly disintegrable tablet according to claim 1, **characterised in that** it is produced by mixing the drug and the sugar alcohol to produce a mixture, subjecting the mixture to wet granulation to produce granules, mixing the crystalline cellulose with the granules and then compression-moulding the granules mixed with the crystalline cellulose.

6. Intraorally rapidly disintegrable tablet according to any of claims 1 to 5, **characterised in that** the sugar alcohol is at least one selected from the group consisting of mannitol, erythritol and xylitol.

7. Intraorally rapidly disintegrable tablet according to any of claims 1 to 5, **characterised in that** the sugar alcohol is mannitol.

8. Intraorally rapidly disintegrable tablet according to any of the previous claims, **characterised in that** the amount of drug that it contains, in relation to one part by weight of the powdered mixture of crystalline cellulose and sugar alcohol, is 0.0001 to 2 parts by weight, preferably 0.005 to 0.1 parts by weight, in particular 0.001 to 0.01 parts by weight.

9. Intraorally rapidly disintegrable tablet according to any of the previous claims, **characterised in that** the drug is selected from antibiotics, chemotherapy drugs, hypnotic and anxiolytic sedatives, anti-epileptic drugs, analgesic, antipyretic and anti-inflammatory agents, drugs for the treatment of Parkinson's, psychotropic drugs, musculoskeletal relaxants, drugs that act on the autonomic nervous system, spasmolytic drugs, cardiotonic drugs, anti-arrhythmic drugs, diuretics, hypotensive agents, capillary protectors, vasoconstrictors, vasodilators, anti-hyperlipidaemic drugs, antitussive expectorants, bronchodilators, anti-diarrhoea drugs, drugs for intestinal function, for peptic ulcers, for the stomach, antacids, cholagogues, gastrointestinal drugs, vitamins, nutritional supplements, drugs for liver disease, anti-gout drugs, anti-diabetic drugs, anti-tumour drugs, anti-histamines, pharmaceutical formulations and drugs for osteoporosis.
